# EUROPEAN PATENT APPLICATION

(11) **EP 0 864 309 A2**
(43) Date of publication of application: **16.09.1998**
(21) Application number: 98301814.4
(22) Date of filing: 10.03.1998
(51) Int. Cl.: A61F 7/00

(54) **Apparatus for cooling human scalps**

(30) Priority: 11.03.1997 GB 9704957
(71) Applicant: Paxman, Neil Eric, Huddersfield, West Yorkshire (GB); Paxman, Glenn Alan, Huddersfield, West Yorkshire (GB); Paxman, Paul Anthony, Batley, West Yorkshire (GB)
(72) Inventor: Paxman, Neil Eric, Huddersfield, West Yorkshire (GB); Paxman, Glenn Alan, Huddersfield, West Yorkshire (GB); Paxman, Paul Anthony, Batley, West Yorkshire (GB)
(74) Representative: Lister, David Anthony

(57) **Abstract**

Known kinds of apparatus for cooling human scalps, especially though not exclusively for preventing or reducing hair loss in conjunction with anti-cancer treatment by chemotherapy, suffer for various reasons from insufficiently accurate temperature control which drastically reduces success rates. All but the most basic and ineffective kinds of such apparatus are also very large units. Portable apparatus with accurate temperature control comprises a glycol tank 40 of minimal capacity, a generally conventional refrigeration system including an evaporator 42 disposed within the tank, a thermostat 76 for controlling the system to maintain the temperature of the glycol within close limits, and a single cooling hat 24 through which glycol from the tank is continuously circulated by a pump 66. The thermostat 76 is disposed at the outlet of the tank; and the pump continuously circulates some glycol directly back to the tank by way of a by-pass pipe 72 without passing through the hat. A safety device 82 which warns of low coolant flow without shutting down the apparatus comprises a temperature measuring probe arranged to activate an audible and/or visual alarm and disposed adjacent to the tank in a pipe 22 returning glycol from the hat to the tank.

## Description

This invention relates to apparatus for cooling human scalps, particularly but not exclusively in conjunction with the taking of anti-cancer drugs.

It is known that in the case of at least some chemotherapy patients, hair loss can be prevented or reduced by inducing scalp hypothermia.

All the scalp cooling means which are currently available have disadvantages which will now be set out.

Simple ice-packed hats or helmets have no temperature control at all.

Static cold air cooling units are very expensive and suffer from temperature fluctuations depending on ambient temperature. Such a unit has the facility to cool two heads at once, each cooling hat having a thermostat attached to the patient's head. Unless the unit is provided with two pumps, two compressors or a magnetic valve system, temperature fluctuations will occur. It is also a very large unit.

Units supplying a flow of cooled glycol through a rubber hat also have the facility to cool two heads at once but because of the relatively large amount of glycol employed, typically around eight litres or more, relative to the power of the refrigeration system, compounded by unsuitable positioning in the glycol tank of the coolant temperature control thermostat, fluctuations in temperature occur in the glycol and have a detrimental effect on patient success rates. Again this is a very large unit.

Treating two patients at once has the inherent disadvantage that the head of a large person requires appreciably more cooling than that of a small person. However, accurate temperature control is critical for good results.

The object of the present invention is to avoid or mitigate the aforesaid disadvantages, and especially to provide accurate temperature control.

According to the invention, apparatus for cooling human scalps comprises a liquid coolant tank of minimal capacity, a refrigeration system including an evaporator which is supplied with cold refrigerant gas and constitutes a heat exchanger disposed within the tank, a thermostat for controlling the refrigeration system so as to maintain the temperature of the coolant within close limits, and a single cooling hat through which coolant from the tank is continuously circulated by a pump.

Preferably, the thermostat is disposed at the outlet of the tank.

Preferably, also, the pump continuously circulates some coolant directly back to the tank by way of a by-pass pipe without passing through the hat.

Preferably, a safety device which warns of low coolant flow without shutting down the apparatus comprises a temperature measuring probe disposed adjacent to the tank in a pipe returning coolant from the hat to the tank.

Preferably, also, the probe is arranged to activate an audible and/or visual alarm.

One embodiment of the invention will now be described, by way of example only, with reference to the accompanying drawings of which:-
Figure 1 is a perspective view of a complete scalp cooling unit mounted on a trolley; and
Figure 2 is a plan view of a refrigeration system and a coolant tank and pump forming the principal operational parts of the unit.

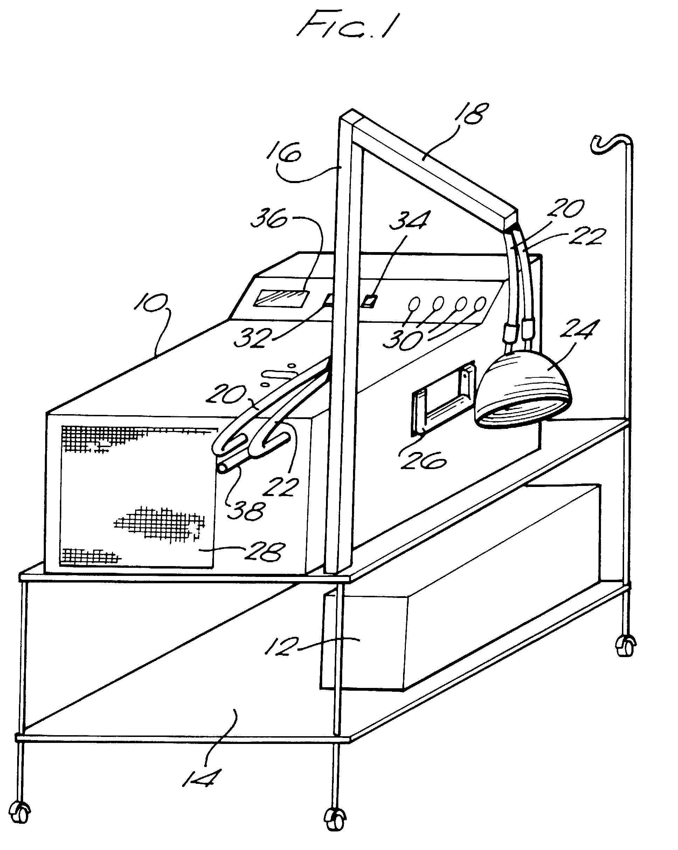

Referring now to Figure 1, apparatus for cooling human scalps includes a cooling unit 10 and a power pack 12 mounted on a wheeled trolley 14 which also carries a vertical post 16 with a radial arm 18 fixed to its upper end. The post 16 and arm 18 support flexible coolant supply and return pipes 20 and 22 respectively for circulating coolant from the unit 10 through a single rubber cooling hat or helmet 24 to be worn on a patient's head. Differently-sized hats have connectors enabling them to be selectively fitted to the pipes 20 and 22. The unit 10 includes two carrying handles one of which is shown at 26, an air outlet grille 28 hereinafter referred to and an air inlet grille (not shown) at its other end, a number of fuses 30, a power switch 32, a pump switch 34, a coolant temperature control thermostat having a read-out 36 of liquid crystal diode type, a safety thermostat, and a coolant overflow pipe 38.

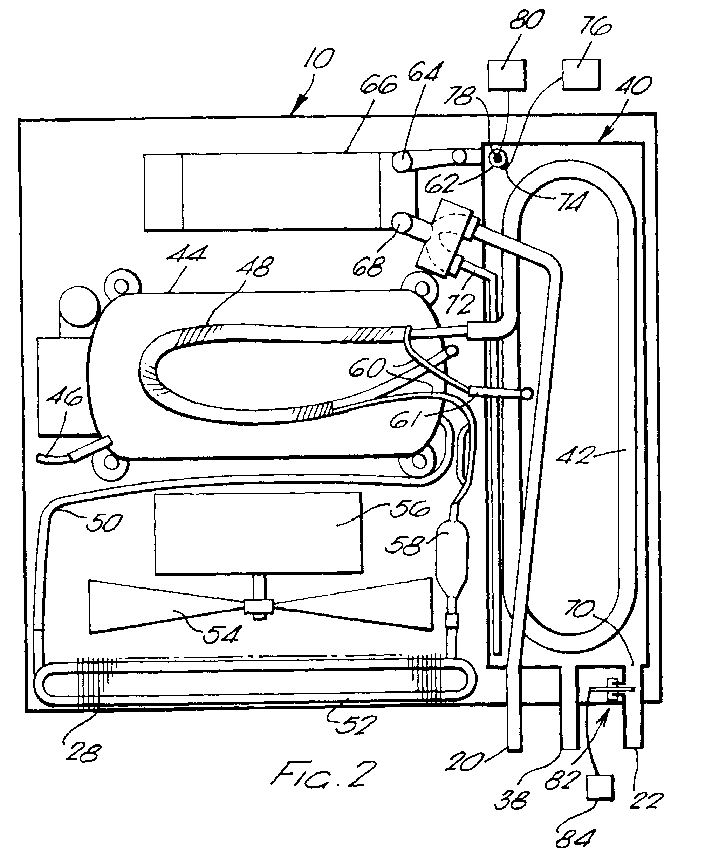

Referring now to Figure 2, the cooling unit 10 comprises essentially a liquid coolant tank 40 of minimal capacity, say two litres, which contains glycol, and a generally conventional refrigeration system including an evaporator 42 which is disposed within the tank 40. More specifically, the refrigeration system comprises a motorised compressor 44 having a charging point 46, a suction line 48, and a discharge line 50 through which liquid refrigerant (which can be of any suitable type) is passed to a condenser 52 with an associated fan 54 driven by a motor 56 which draws air in through the grille 28 and then through the condenser 52 (see Figure 1) to supercool the liquid refrigerant. The refrigerant next passes through a dryer 58 and then through a capillary tube 60 coiled around the suction line 48 which makes it relatively hot. The capillary tube 60 discharges the refrigerant as a cold gas at 61 into the evaporator 42, which consists of loops of 22 millimetre diameter copper tube traversing the whole interior of the tank 40 and then leading to the suction line 48.

The glycol in the tank 40 is cooled by heat exchange with the evaporator 42 and is then withdrawn through a tank outlet 62 to an inlet port 64 of a motorised pump 66 which circulates it from an outlet port 68 through the supply pipe 20, the hat 24 and the return pipe 22 back to a tank inlet 70. The port 68 also communicates with a restricted by-pass pipe 72 which is adapted to return glycol continuously to the tank 40 so as to prevent damage to the pump 66 if said pump is wrongly left running when the hat 24 is disconnected, and also to increase the circulation of glycol through the tank 40 so as advantageously to minimise any tendency for hot spots to develop therein. The coolant overflow pipe 38 communicates with the tank 40. A probe 74 for the coolant temperature control thermostat 76, and a probe 78 for the safety thermostat 80, are disposed at the tank outlet 62.

The motorised compressor 44, the fan motor 56 and the motorised pump 66 are all normally powered by mains electricity, but the power pack 12 contains batteries (not shown) which take over automatically if there is a power failure or if the patient wishes to visit the toilet or simply take a short walk accompanied by the trolley 14 without interrupting the treatment which may last for several hours.

In operation, the pump 66 continuously circulates cooled glycol through the hat 24 in order to induce hypothermia in the scalp of a patient wearing the hat. Temperature variations are held to less than 1 degree Celsius on either side of a desired temperature setting, regardless of ambient temperature, by intermittent operation of the refrigeration unit under control of the thermostat 76. These close limits are achieved by virtue of the minimal amount of glycol employed relative to the power of the refrigeration system aided by the disposition of the probe 74 of the coolant temperature control thermostat 76 at the tank outlet 62. The safety thermostat 80 prevents the refrigeration system from evaporating the refrigerant at less than -8 degrees Celsius. The apparatus is approximately one-third the size of the known units which supply a flow of cooled glycol or employ static cold air cooling, thus enabling it to be readily moveable around on the trolley 14 together with the patient as hereinbefore stated. The invention effectively senses individual heat loss from the head of any size of patient. It might possibly be effective also in the remedial treatment of certain types of hair loss not due to the taking of anti-cancer drugs, where the follicles are not dead. Suitable liquid coolants other than glycol can equally well be employed.

In a highly desirable modification, the apparatus is provided with a safety device comprising a probe 82 and a thermostatic controller 84 shown in Figure 2 of the drawings which warns of low coolant flow and consequent deleterious coolant temperature rise, due either to flow restriction, typically caused by kinking of one or both of the flexible pipes 20 and 22, or to low coolant level. This safety device does not require an expensive flow rate sensor, but comprises a temperature measuring probe at a point in the return pipe 22 adjacent to the tank 40 and within the confines of the unit 10. A T-piece is incorporated into the pipe 22 at said point to enable said probe to be inserted in sealed fashion into the return flow of the coolant. The arrangement is such that if the temperature of the coolant rises by more than say 3 degrees Celsius above the preferred temperature, the probe activates an audible and/or visual alarm which warns of flow restriction or low coolant level but does not shut down the apparatus.

## Claims

1. Apparatus for cooling human scalps comprising a liquid coolant tank (40) of minimal capacity, a refrigeration system including an evaporator (42) which is supplied with cold refrigerant gas and constitutes a heat exchanger disposed within the tank, a thermostat (76) for controlling the refrigeration system so as to maintain the temperature of the coolant within close limits, and a single cooling hat (24) through which coolant from the tank is continuously circulated by a pump (66).

2. Apparatus according to claim 1, wherein the thermostat (76) is disposed at the outlet of the tank (40).

3. Apparatus according to either of the preceding claims, wherein the pump (66) continuously circulates some coolant directly back to the tank (40) by way of a by-pass pipe (72) without passing through the hat (24).

4. Apparatus according to any one of the preceding claims, wherein a safety device (82) which warns of low coolant flow without shutting down the apparatus comprises a temperature measuring probe disposed adjacent to the tank (40) in a pipe (22) returning coolant from the hat (24) to the tank.

5. Apparatus according to claim 4, wherein the probe is arranged to activate an audible and/or visual alarm.

6. Apparatus for cooling human scalps constructed, arranged and adapted to operate substantially as hereinbefore described with reference to, and as illustrated by, Figure 2 of the accompanying drawings.

7. Apparatus for cooling human scalps constructed, arranged and adapted to operate substantially as hereinbefore described with reference to, and as illustrated by, Figures 1 and 2 of the accompanying drawings.
